# EUROPÄISCHE PATENTANMELDUNG

(11) **EP 1 374 838 A1**
(43) Veröffentlichungstag der Anmeldung: **02.01.2004**
(21) Anmeldenummer: 03012689.0
(22) Anmeldetag: 04.06.2003
(51) Int. Cl.: A61K 7/11

(54) **Cremiger Haarfestiger Decylglucosid enthaltend**

(30) Priorität: 22.06.2002 DE 10227868
(71) Anmelder: Beiersdorf AG, 20245 Hamburg (DE)
(72) Erfinder: Detert, Marion, 22455 Hamburg (DE); Sass, Viola, 22880 Wedel (DE); Franck, Kerstin, 22529 Hamburg (DE); Primmel, Bettina, 20146 Hamburg (DE)

(57) **Zusammenfassung**

Schäumbarer Haarfestiger enthaltend:
a) ein oder mehrere anionische Polymere,
b) ein oder mehrere kationische Polymere,
c) Decylglucosid,
d) Wasser,
neben gegebenenfalls weitere kosmetischen Wirk-, Hilfs- und Zusatzstoffen.

## Beschreibung

Die vorliegende Erfindung betrifft Haarfestiger enthaltend Decylglucosid.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Natürliches Haar hängt meist schlaff und ohne Volumen vom Kopf herab. Ein Ziel der Haarpflege ist es daher, dem Haar Fülle und Frisur zu geben. Zur temporären Verformung des Haares und Gestaltung (engl. Styling) vielseitiger Frisuren verwendet man Haarfestiger, meist in Form von Schaumfestigern oder Haarsprays. Beide haarkosmetischen Mittel sind in Ihrer Zusammensetzung ähnlich unterscheiden sich aber in ihrer Aufgabe und Anwendung. Schaumfestiger (auch Haarschaumfestiger genannt) werden in der Regel zur Frisurgestaltung im feuchten Haar verteilt, Haarsprays zur Fixierung auf die (trockene) fertig gestylte Frisur gesprüht. Neben den wegen ihrer leichten und angenehmen Anwendbarkeit zunehmend beliebten Schaumhaarfestigern und flüssigen Haarfestigern werden auch Haarfestigergele angeboten.

Die beiden Grundbausteine von Haarfestigern sind die Filmbildner und das Lösungsmittel. Als Filmbildner werden meist Mischpolymerisate (Polymere) aus Vinylpyrrolidon und Vinylacetat oder neutralisierter Crotonsäure eingesetzt. Auch andere kationische Polymere (d.h. in der Regel Polymere mit quaterem Stickstoffatomen) sowie anionische Polymere (z.B. Polymere mit Carboxylat-Gruppen) können als Filmbildner eingesetzt werden. Filmbildner bleiben nach dem Verdunsten des Lösungsmittels (meist Wasser und/oder Ethanol) als Film auf dem Haar und fixieren dessen Form. Haarschaumfestiger enthalten zusätzlich Treibmittel wie Propan, n-Butan und/oder Isobutan.

Je nach gewünschter Festigkeit der Frisur werden unterschiedlich stark festigende Filmbildner in unterschiedlichen Mengenanteilen eingesetzt. Man unterscheidet daher Haarfestiger mit schwacher, normaler und starker Festigung sowie Festiger für normales, trockenes und fettiges Haar [W. Umbach (Hrsg.): Kosmetik, Entwicklung, Herstellung und Anwendung kosmetischer Mittel, 2. Aufl., Thieme Verlag, Stuttgart, 1995]. Für stark festigende Haarfestiger werden in der Regel Kombinationen aus kationischen und anionischen Polymeren als Filmbildner verwendet.

Herkömmliche Haarfestiger, die anionische und kationische Polymere enthalten, weisen jedoch eine Reihe von Nachteilen und Unzulänglichkeiten auf. Ihre Schaumcharakteristik (u.a. Stabilität, Verteilbarkeit im Haar, etc.) ist in der Regel unzureichend. Ihr Schaum hat eine sehr feste Konsistenz und fühlt sich wenig cremig an. Schon beim Verteilen des Schaumes in den Händen wird dieser sehr flockig. Der Schaum läßt sich schlecht im Haar verteilen, wodurch das Haar ein ungleichmäßiges und reduziertes Volumen erhält.

Es war daher die Aufgabe der vorliegenden Erfindung, den Nachteilen des Standes der Technik abzuhelfen und Haarfestiger zu entwickeln, deren sensorische Eigenschaften und Schaumcharakteristik denen herkömmlicher Produkte überlegen ist.

Überraschend wird die Aufgabe gelöst durch einen schäumbaren Haarfestiger enthaltend:
a) ein oder mehrere anionische Polymere,
b) ein oder mehrere kationische Polymere,
c) Decylglucosid,
d) Wasser,
neben gegebenenfalls weitere kosmetischen Wirk-, Hilfs- und Zusatzstoffen.

Zwar sind Decylglucoside in kosmetischen Haarzubereitungen an sich bekannt. So beschreibt beispielsweise die DE 69324099 T2 Shampoos und Spühlungen, die DE 19907468 A1 Emulsionen oder die DE 19855097 A1 Färbemittel mit einem Gehalt an Decylglucosiden. Doch konnten diese Schriften nicht den Weg zur vorliegenden Erfindung weisen.

Die erfindungsgemäßen Zubereitungen zeichnen sich im aufgeschäumten Zustand durch einen cremigen, pflegend anmutenden, gut in der Hand und im Haar verteilbaren Schaum aus, ohne das dessen Festigungsleistung reduziert wird. Der Schaum flockt nicht beim Verreiben, die im Haar verteilte Zubereitung verleiht diesem eine hohe Festigkeit und Volumen.

Es ist erfindungsgemäß vorteilhaft, die anionischen Polymere in einer Konzentration von 0,01 bis 20 Gewichts-%, bevorzugt in einer Konzentration von 0,1 bis 10 Gewichts-% und ganz besonders bevorzugt in einer Konzentration von 0,25 bis 2,5 Gewichts-%, jeweils bezogen auf das Gesamtgewicht der Zubereitung in dem schäumbaren Haarfestiger einzustzen.

Außerdem ist es erfindungsgemäß vorteilhaft, die kationischen Polymere in einer Konzentration von 0,01 bis 50 Gewichts-%, bevorzugt in einer Konzentration von 0,1 bis 10 Gewichts-% und ganz besonders bevorzugt in einer Konzentration von 0,25 bis 2,5 Gewichts-%, jeweils bezogen auf das Gesamtgewicht der Zubereitung in dem schäumbaren Haarfestiger einzusetzen.

Ferner ist es erfindungsgemäß vorteilhaft, Decylglucosid in einer Konzentration von 0,01 bis 5 Gewichts-%, bevorzugt in einer Konzentration von 0,025 bis 3 Gewichts-% und ganz besonders bevorzugt in einer Konzentration von 0,05 bis 1,5 Gewichts-%, jeweils bezogen auf das Gesamtgewicht der Zubereitung in dem schäumbaren Haarfestiger einzusetzen.

Bei dem erfindungsgemäßen Decylglucosid (INCI: Decyl Glucoside) handelt es sich um ein Zuckertensid, welches aus Decylalkohol und Glucosid-Polymeren gebildet werden kann und bisher als Feuchthaltemittel (engl. humectant) oder oberflächenaktive Verbindung eingesetzt wird. Erfindungsgemäß besonders bevorzugt ist beispielsweise das Decylglucosid Plantacare 2000® der Fa. Cognis.

Erfindungsgemäß vorteilhafte anionische Polymere sind Homo- oder Copolymere mit Säuregruppen enthaltenden Monomereinheiten, welche gegebenenfalls mit Comonomeren, die keine Säuregruppen enthalten copolymerisiert sind. Geeignete Monomere sind ungesättigte, radikalisch polymerisierbare Verbindungen, welche mindestens eine Säuregruppe besitzen, insbesondere Carboxyvinylmonomere. Geeignete Säuregruppen enthaltende Monomere sind beispielsweise Acrylsäure, Methacrylsäure, Crotonsäure, Maleinsäure bzw. Maleinsäureanhydrid oder deren Monoester, Aldehydcarbonsäuren oder Ketocarbonsäuren.
Nicht mit Säuregruppen substituierte Comonomere sind beispielsweise Acrylamid, Methacrylamid, Alkyl- und Dialkylacrylamid, Alkyl- und Dialkylmethylacrylamid, Alkylacrylat, Alkylmethacrylat, Vinylcaprolactam, Vinylpyrrolidon, Vinylester, Vinylalkohol, Propylenglycol oder Ethylenglycol, aminsubstituierte Vinylmonomere wie z.B. Dialkylaminoalkylacrylat, Dialkylaminoalkylmethacrylat, Monoalkyl-aminoalkylacrylat und Monoalkylaminoalkylmethacrylat.
Bevorzugte Polymere mit Säuregruppen sind insbesondere Copolymere der Acrylsäure oder Methacrylsäure mit Monomeren ausgewählt aus Acrylsäure- oder Methacrylsäureestern, Acrylamiden, Methacrylamiden und Vinylpyrrolidonen, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden.
Weiter geeignete Polymere sind: vernetzte oder unvernetzte Vinylacetat/ Crotonsäure Copolymere (INCI VA/Crotonates Copolymer) z.B. Resyn 28-1310 von National Starch oder Luviset CA66 von BASF; Terpolymere aus Vinylacetat, Crotonat und Vinylalkanoat, insbesondere Vinylacetat/Crotonat/vinylnoedecanoat Copolymere (lNCl: VA/Crotonates/Vinylneodecanoate Copolymer) z.B. Resyn 28-2930 von National Starch; partialveresterte Copolymere zwischen Vinylmethylether und Maleinsäureanhydrid (lNCl: Ethyl-, lsopropyl-,Butylester of PVM/MA Copolymer) z.B. Gantrez ES 225 oder Gantrez ES 425 von ISP; Copolymere aus Acrylsäure oder Methacrylsäure mit Alkylacrylaten und/oder N-Alkylacrylamiden, insbesondere Copolymere aus Methacrylsäure und Alkyacrylaten sowie Terpolymeren aus Acrylsäure, Alkylacrylaten und N-Alkylacrylamiden wie Acrylsäure/Ethylacrylat/N-tert.-Butylacrylamid Terpolymer (INCI: Acrylate/Acrylamide Copolymer) z.B. Ultrahold 8 von BASF oder tert.-Butylacrylat/Ethylacrylat/ Methacrylsäure Terpolymer (INCI: Acrylates Copolymer), z.B. Luvimer von BASF; Polystyrolsulfonate (lNCl: Sodium Polystyrene Sulfonate) z.B. Flexan 130 von National Starch und Polyester.
Geeignete anionische Polymere sind auch in Wasser lösliche oder dispergierbare anionische Polyurethane, z.B. Luviset PUR von BASF.

Ein erfindungsgemäß besonders bevorzugtes anionisches Polymer stellt mit 2-Amino-2-methyl-1-propanol neutralisiertes Acrylat/Allylmethacrylat-Copolymer dar, welches nach der INCI mit AMP-Acrylates/Allyl Methacrylate Copolymer bezeichnet wird.

Es ist erfindungsgemäß von Vorteil, wenn als kationische Polymere Polymere mit basischen Gruppen z.B. Copolymere von aminsubstituierten Vinylmonomeren und nicht aminsubstituierten, nicht kationischen Monomeren eingesetzt werden. Aminsubstituierte Vinylmonomere sind z.B.Dialkylaminoalkylacrylat, Dialkylaminoalkylmethacrylat, Monoalkylaminoalkylacrylat, Monoalkylaminoalkylmethacrylat, wobei als Alkylgruppen dieser Monomere vorzugsweise niedere Alkylgruppen wie z.B. C1-C7- Alkylgruppen, besonders bevorzugt C1- C3-Alkylgruppen eingesetzt werden.
Geeignete ammoniumsubstituierte Vinylmonomere sind z.B. Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium oder Imidazolium, z.B. Alkylvinylpyridinium oder Alkylvinylimidazolium Salze. Die Alkylgruppen dieser Monomere sind vor-zugsweise niedere Alkylgruppen wie z.B. C1-C7- Alkylgruppen, wobei C1- C3-Alkylgruppen besonders bevorzugt sind. Geeignete Polymere sind unter den Bezeichnungen Polyquaternium beschriebenen Polymere wie quaternisierte Copolymere von Vinylimidazol, Vinylpyrrolidon und/oder Vinylcaprolactam (Polyquaternium-16, -44 oder -46), quaternisiertes Vinylpyrrolidon/-Dimethylaminoethylmethacrylat Copolymer (Polyquaternium-11), Homo- und Copolymere von Dimethyldiallylammoniumchlorid (Polyquaternium-6, -7 oder-22), quaternisierte Hydroxyethylcellulose (Polyquaternium-10) oder quaternisierte Guarderivate.
Nicht aminsubstituierte, nichtkationische Comonomere sind z.B. Acrylamid, Methacrylamid, Alkyl- und Dialkylacrylamid, Alkyl- und Dialkylmethacrylamid, Acrylacrylat, Alkylmethacrylat, Vinylcaprolacton, Vinylpyrrolidon, Vinylester, Vinylalkohol, Maleinsäreanhydrid, Propylenglycol oder Ethylenglycol, wobei die Alkylgruppen dieser Monomere vorzugsweise aus niederen Alkylgruppen wie z.B. C1-C7- Alkylgruppen und besonders bevorzugt C1- C3-Alkylgruppen gebildet werden.
Bevorzugte kationische Polymere sind: quaternisiertes Vinylpyrrolidon/-Dialkylaminoalkylmethacrylat Copolymere, insbesondere quaternisiertes Vinylpyrrolidon/Dimethylaminoethylmethylacrylat Copolymer (INCI: Polyquaternium-11), z.B. Gafquat 755 von ISP oder Luviquat PQ11 von BASF; MethylvinylimidazoliumchloridNinylpyrrolidon Copolymer (INCI: Polyquaternium-16), z.B. die Luviquat Typen FC 370, FC 550, FC 905 oder HM552 von BASF; Methylvinylimidazolium Methylsulfat/Vinylpyrrolidon Copolymer (lNCl: Polyquaternium-44), z.B. Luviquat Care oder Luviquat MS 370 von BASF; oder das Copolymer aus Vinylcaprolactam, Vinylpyrrolidon und quaternisiertem Vinylimidazol (lNCl Polyquaternium-46), z.B. Luviquat Hold von BASF.

Das erfindungsgemäß besonders bevorzugte kationische Polymer ist ein Copolymeres aus Hydroxyethylcellulose und Dimethyldiallylammoniumchlorid welches nach lNCl mit Polyquaternium-4 bezeichnet wird.

Erfindungsgemäß besonders bevorzugt ist die Kombination aus anionischen AMP-Acrylates/Allyl Methacrylate Copolymer (0,01 bis 20 Gewichts%) mit kationischen Polyquaternium-4 (0,01 - 20 Gewichts%).

Die erfindungsgemäßen Haarfestiger können erfindungsgemäß vorteilhaft mit einem Treibgas aufgeschäumt werden. Dieses wird erfindungsgemäß in einer Menge von 0,5 bis 20 Gewichts-%, besonders vorteilhaft in einer Menge von 5 bis 15 und ganz besonders vorteilhaft in einer Menge von 8 bis 11 Gewichts-%, jeweils bezogen auf das Gesamtgewicht der Formulierung eingesetzt. Erfindungsgemäß besonders vorteilhafte Treibgase sind Propan, Isobutan und n-Butan sowie deren Mischungen.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Antioxidanien, Bakterizide, Parfüme, Emulgatoren, weichmachende, anfeuchtende und/oder feuchthaltende Substanzen, Fette, Öle, Wachse, Farbstoffe, Pigmente, die eine färbende Wirkung haben, Verdickungsmittel oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate. Auch die Einarbeitung von UV-Lichtschutzfiltern ist erfindungsgemäß vorteilhaft. Die aufgeführte Liste an Zusatzstoffen soll selbstverständlich nicht limitierend sein.

Eine besonders bevorzugte Ausführungsform der erfindungsgemäßen Haarfestiger ist ihr Vorliegen in Form einer wässrig und/oder alkoholischen Lösung.

Die erfindungsgemäßen Haarfestiger werden erfindungsgemäß vorteilhaft in einer Pumpschäumer- oder Aerosolverpackung als Schaumspender verpackt (aufbewahrt), aus der heraus sie vorteilhaft angewendet werden können.

Auch sind Schaumspender auf Pumpschäumer- oder Aerosolverpackungsbasis, welche einen erfindungsgemäßen Haarfestiger enthalten, endungsgemäß.

Erfindungsgemäß ist weiterhin die Verwendung erfindungsgemäßen Haarfestigers als Schaumfestiger.

Erfindungsgemäß ist nicht zuletzt die Verwendung von Decylglucosid zur Erhöhung der Cremigkeit und Verbesserung der Verteilbarkeit des Schaums von schäumbaren Haarfestigern.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispielrezepturen** | | | | | |
|---|---|---|---|---|---|
| | **1** | **2** | **3** | **4** | **5** |
| AMP-Acrylates/Allyl Methacrylate Copolymer | 2,0 | 3,0 | - | - | 3,0 |
| Acrylates Copolymer | - | - | 2,5 | 3,0 | - |
| Polyquaternium-4 | 1,0 | 0,5 | - | - | 1,0 |
| Polyquaternium-16 | - | - | 2,0 | 1,0 | - |
| Decylglucosid | 0,1 | 0,15 | 0,15 | 0,15 | 0,2 |
| Cetyltrimethylammoniumchlorid | 0,2 | 0,25 | 0,25 | 0,25 | 0,20 |
| Parfüm, Lösungsvermittler | q.s. | q.s. | q.s. | q.s. | q.s. |
| Konservierungsmittel | 0,3 | - | 0,3 | - | 0,3 |
| Treibmittel | 10,0 | 10,0 | 10,0 | 10,0 | - |
| Alkohol | - | 10,0 | - | 10,0 | - |
| Wasser | ad 100 | ad 100 | ad 100 | ad 100 | ad 100 |

## Patentansprüche

1. Schäumbarer Haarfestiger enthaltend:
a) ein oder mehrere anionische Polymere,
b) ein oder mehrere kationische Polymere,
c) Decylglucosid,
d) Wasser,
neben gegebenenfalls weitere kosmetischen Wirk-, Hilfs- und Zusatzstoffen.

2. Schäumbarer Haarfestiger nach Anspruch 1, **dadurch gekennzeichnet, dass** dieser anionische Polymere in einer Konzentration von 0,01 bis 20 Gewichts-%, bezogen auf das Gesamtgewicht der Zubereitung enthält.

3. Schäumbarer Haarfestiger nach einem der Ansprüche 1 oder 2, **dadurch gekennzeichnet, dass** dieser kationische Polymere in einer Konzentration von 0,01 bis 20 Gewichts-%, bezogen auf das Gesamtgewicht der Zubereitung enthält.

4. Schäumbarer Haarfestiger nach einem der Ansprüche 1 bis 3, **dadurch gekennzeichnet, dass** dieser Decylglucosid in einer Konzentration von 0,01 bis 5 Gewichts-%, bezogen auf das Gesamtgewicht der Zubereitung enthält.

5. Schäumbarer Haarfestiger nach einem der Ansprüche 1 bis 4, **dadurch gekennzeichnet, dass** als anionisches Polymer mit 2-Amino-2-methyl-1-propanol neutralisiertes Acrylat/Allylmethacrylat-Copolymer (INCI: mit AMP-Acrylates/Allyl Methacrylate Copolymer) eingesetzt wird.

6. Schäumbarer Haarfestiger nach einem der Ansprüche 1 bis 5, **dadurch gekennzeichnet, dass** als kationisches Polymer ein Copolymeres aus Hydroxyethylcellulose und Dimethyldiallylammoniumchlorid (lNCl: Polyquaternium-4 eingesetzt wird.

7. Schäumbarer Haarfestiger nach einem der Ansprüche 1 bis 6, **dadurch gekennzeichnet, dass** dieser mit einem Treibgas aufgeschäumt wird.

8. Schäumbarer Haarfestiger nach einem der Ansprüche 1 bis 7, **dadurch gekennzeichnet, dass** der Haarfestiger in einem Pumpspender oder einer Aerosoldose aufbewahrt und aus dieser heraus angewendet wird.

9. Verwendung des schäumbaren Haarfestigers nach einem der vorhergehenden Ansprüche als Schaumfestiger.

10. Verwendung von Decylglucosid zur Erhöhung der Cremigkeit und Verbesserung der Verteilbarkeit des Schaums von schäumbaren Haarfestigern nach einem der vorhergehenden Ansprüche.
